# EUROPÄISCHE PATENTSCHRIFT

(11) **EP 2 736 550 B1**
(45) Veröffentlichungstag und Bekanntmachung des Hinweises auf die Patenterteilung: **27.07.2016**
(21) Anmeldenummer: 12751456.0
(22) Anmeldetag: 27.07.2012
(51) Int. Cl.: A61M 1/00

(54) **VORRICHTUNG ZUM ABSAUGEN VON FLÜSSIGKEITEN UND/ODER PARTIKELN AUS KÖRPERÖFFNUNGEN**
DEVICE FOR SUCTION OF LIQUIDS AND/OR PARTICLES FROM OPENINGS OF THE BODY
DISPOSITIF PERMETTANT D'ASPIRER DES LIQUIDES ET/OU DES PARTICULES ISSUS D'OUVERTURES CORPORELLES

(30) Priorität: 27.07.2011 DE 102011052196
(43) Veröffentlichungstag der Anmeldung: 04.06.2014
(73) Patentinhaber: MAQUET GmbH, 76437 Rastatt (DE)
(72) Erfinder: GINZINGER, Lena, 76530 Baden-Baden (DE); HARLACHER, Peter, 77815 Bühl (DE)
(74) Vertreter: Zacco GmbH
(86) Internationale Anmeldenummer: PCT/EP2012/064803
(87) Internationale Veröffentlichungsnummer: WO 2013/014278

(56) Entgegenhaltungen:
- EP-A1- 1 679 570
- EP-A2- 0 467 425
- US-A- 5 423 780
- US-A- 5 531 712
- US-A1- 2003 216 690

## Beschreibung

Die Erfindung betrifft eine Vorrichtung zum Absaugen von Flüssigkeiten und/oder Partikeln aus Körperöffnungen, die einen Absaugkanal umfasst, durch den die Flüssigkeit und/oder die Partikel absaugbar sind. Ferner hat die Vorrichtung eine Vakuumpumpe zum Erzeugen eines Unterdrucks in dem Absaugkanal, wobei durch diesen Unterdruck die Flüssigkeit und/oder die Partikel abgesaugt werden und der Absaugkanal eine Öffnung zum Zuführen von Nebenluft zum Einstellen des Unterdrucks aufweist. Ferner hat die Vorrichtung eine Nebenlufteinstelleinheit zum Einstellen des Volumenstroms der dem Absaugkanal zugeführten Nebenluft und eine manuell betätigbare Verstelleinheit zum Einstellen der Nebenlufteinstelleinheit.

Die Vorrichtung wird insbesondere in Kliniken, Arztpraxen, Pflegeheimen und/oder bei der Heimpflege eingesetzt, um beispielsweise Blut oder andere Flüssigkeit inklusive der darin enthaltenen Partikel oder Knochensplitter bei Operationen aus natürlichen und/oder künstlichen Körperöffnungen zu entfernen. Die Vorrichtung wird häufig auch als Sekretsauger bezeichnet.

Um die Saugleistung des Saugers an den jeweiligen Anwendungsfall anzupassen, kann die Saugleistung gesteuert werden, indem der Unterdruck variiert werden kann. Hierzu wird dem Absaugkanal Nebenluft zugeführt, wobei die Menge der zugeführten Nebenluft bei bekannten Saugern über ein Nadelventil eingestellt werden kann. Zum manuellen Verstellen dieses Nadelventils ist an einer Bedienoberfläche des Saugers ein Drehknopf vorhanden, wobei durch ein Drehen dieses Knopfes das Nadelventil verstellt wird und somit ebenfalls der Volumenstrom der Nebenluft verändert wird. Aufgrund der überproportionalen Kennlinie des Nadelventils und dem nicht-linearen funktionalem Zusammenhang zwischen dem zugeführten Volumenstrom an Nebenluft und dem sich ergebenden Unterdruck muss bei einem relativ geringen Unterdruck für die gleiche Veränderung des Unterdrucks der Drehknopf um einen wesentlich größeren Winkelbereich als zum Einstellen der gleichen Veränderung des Unterdrucks in einem hohen Vakuumbereich gedreht werden.

Problematisch hieran ist, dass dies zu einem geringen Bedienkomfort führt, da die Bedienperson über das Vakuummeter zum Anzeigen des aktuellen Unterdrucks immer genau darauf achten muss, welcher Unterdruck gerade herrscht, und eine intuitive Bedienung schwer möglich ist.

Aus dem Dokument US 5,423,780 A ist eine Vorrichtung zum Absaugen von Flüssigkeiten und/oder Partikeln aus Körperöffnungen bekannt, bei der ein Verstellen einer Verstelleinheit mit jeweils einem gleichen voreingestellten Verstellweg jeweils die gleiche Änderung des Unterdrucks bewirkt.

Weitere Vorrichtungen zum Absaugen von Flüssigkeiten und/oder Partikeln aus Körperöffnungen sind aus den Dokumenten EP 0 467 425 A2, US 5,531,712 A und US 2003/216690 A1 bekannt.

Es ist Aufgabe der Erfindung, eine Vorrichtung zum Absaugen von Flüssigkeiten und/oder Partikeln aus Körperöffnungen anzugeben, deren Saugleistung auf einfache Weise einstellbar ist.

Diese Aufgabe wird durch eine Vorrichtung mit den Merkmalen des Anspruchs 1 gelöst. Vorteilhafte Weiterbildungen der Erfindung sind in den abhängigen Ansprüchen angegeben.

Erfindungsgemäß sind die Verstelleinheit und die Nebenlufteinstelleinheit derart ausgebildet, dass zumindest im Bereich zwischen einem unteren Grenzwert des Unterdrucks und einem oberen Grenzwert des Unterdrucks das Verstellen der Vertelleinheit um jeweils einen gleichen voreingestellten Verstellweg jeweils die gleiche Änderung des Unterdrucks bewirkt. Hierdurch wird eine besonders einfache Bedienung der Vorrichtung beim Einstellen des Unterdrucks erreicht, da die Bedienperson für die gleiche Änderung des Volumenstroms der Nebenluft, und somit für die gleiche Änderung des zum Absaugen verwendeten Unterdrucks, jeweils die Verstelleinheit um den gleichen Verstellweg verstellen muss.

Unter dem im Absaugkanal herrschenden Unterdruck wird derjenige Unterdruck verstanden, der sich ergibt, wenn die Öffnung des Absaugkanals, durch die die Flüssigkeit und/oder die Partikel eingesaugt werden, verschlossen ist. Somit wird unter dem im Absaugkanal herrschenden Unterdruck derjenige Unterdruck verstanden, der bei der jeweiligen Stellung der Nebenlufteinstelleinheit maximal erzeugbar ist.

Der untere Grenzwert des Unterdrucks gibt denjenigen Unterdruck an, der mit Hilfe der Vorrichtung minimal erzeugbar ist. Entsprechend ergibt der obere Grenzwert des Unterdrucks denjenigen Unterdruck an, der mit Hilfe der Vorrichtung maximal erzeugbar ist. Der obere Grenzwert hat insbesondere einen Wert zwischen -70 kPa und -90 kPa, vorzugsweise von etwa -80 kPa. Der untere Grenzwert hat insbesondere einen Wert von 0 kPa. Die entsprechenden Unterdrücke beziehen sich vorzugsweise auf den jeweils herrschenden atmosphärischen Druck.

Bei der Verstelleinheit handelt es sich insbesondere um einen drehbaren Knopf oder Ring, so dass der Verstellweg einer Winkeldrehung entspricht. Somit ist zum Ändern des Unterdrucks um einen gleichen Wert jeweils der gleiche Drehwinkel zu verstellen. Alternativ kann die Verstelleinheit derart ausgebildet sein, dass sie einen linear verschiebbaren Schieber umfasst, wobei in diesem Fall für die gleiche Änderung des Unterdrucks der Schieber jeweils um den gleichen Weg verschoben werden muss.

Die Nebenlufteinstelleinheit kann insbesondere derart ausgebildet sein, dass mit ihrer Hilfe die Öffnung des Absaugkanals, durch die die Nebenluft zugeführt wird, verschließbar ist. In diesem Fall wird der Strömungsquerschnitt des Volumenstroms der Nebenluft dadurch verändert, dass diese Öffnung teilweise oder ganz verschlossen wird. Unter dem Strömungsquerschnitt wird derjenige Querschnitt verstanden, der dem Volumenstrom der Nebenluft beim Zuführen zu dem Absaugkanal zur Verfügung steht. Alternativ kann die Nebenlufteinstelleinheit derart angeordnet sein, dass mit ihrer Hilfe der Querschnitt eines Nebenluftkanals, durch den die Nebenluft zu der Öffnung geführt wird, teilweise oder vollständig verschließbar ist.

Durch Änderung des Strömungsquerschnitts des Volumenstroms der Nebenluft ändert sich auch die pro Zeiteinheit zugeführte Menge an Nebenluft und somit der in dem Absaugkanal herrschende Unterdruck. Dies wiederum bewirkt, dass die Absaugleistung, mit der die Flüssigkeit und/oder die Partikel aus der Körperöffnung abgesaugt werden, sich entsprechend verändert und somit auf einfache Weise einstellbar ist.

Bei einer bevorzugten Ausführungsform ist die Verstelleinheit stufenweise verstellbar, wobei zumindest im Bereich zwischen dem unteren Grenzwert und dem oberen Grenzwert das Verstellen der Verstelleinheit um jeweils eine Stufe jeweils die gleiche Änderung des Unterdrucks bewirkt. Die Verstelleinheit ist hierzu insbesondere derart ausgebildet, dass sie entsprechend der Stufe einrastet. Alternativ kann die Verstelleinheit auch kontinuierlich verstellbar sein.

Die Nebenlufteinstelleinheit weist insbesondere ein Ventil auf, mit dessen Hilfe der Strömungsquerschnitt des Volumenstroms der Nebenluft veränderbar ist. Somit kann, ohne eine aufwändige Kupplung zwischen der Verstelleinheit und dem Ventil auf einfache Weise ein entsprechender linearer Zusammenhang zwischen dem Verstellweg der Verstelleinheit und der Änderung des Unterdrucks erreicht werden.

Ferner kann die Nebenlufteinstelleinheit eine Blende zum Verschließen mindestens eines Teilbereiches eines Nebenluftkanals zum Zuführen der Nebenluft und/oder mindestens eines Teilbereichs der Öffnung zum Zuführen der Nebenluft umfassen. Über eine solche Blende ist es besonders einfach möglich, den Unterdruck synchron zum Verstellweg der Verstelleinheit zu verändern. Der Nebenluftkanal und/oder die Öffnung haben hierbei insbesondere einen dreieckigen Querschnitt, wobei die Nebenlufteinstelleinheit eine schneckenförmige Blende umfasst, über die der Querschnitt teilweise oder vollständig verschließbar ist. Somit wird bei einem Drehen der Blende um den gleichen Winkel der Querschnitt jeweils derart verkleinert bzw. vergrößert, dass sich der Volumenstrom der Nebenluft so ändert, dass die gewünschte linear synchrone Kopplung zwischen der Verstelleinheit und dem im Absaugkanal herrschenden Unterdruck ergibt.

Bei einer bevorzugten Ausführungsform ist ein Manometer zum Anzeigen des jeweils aktuellen Unterdrucks vorgesehen, so dass die Bedienperson auf einfache Weise überwachen kann, welcher Unterdruck aktuell in dem Absaugkanal herrscht. Insbesondere kann somit vermieden werden, dass über einen zu großen Unterdruck die Gesundheit des Patienten beeinträchtigt wird.

Das Manometer hat insbesondere eine kreissektorförmige Skala und einen um den Mittelpunkt der kreisförmigen Skala drehbar gelagerten Zeiger zum Anzeigen des aktuellen Unterdrucks auf der Skala. Die Verstelleinheit weist einen Ring auf, dessen Mittelpunkt mit dem Mittelpunkt der Skala zusammenfällt und der um seinen Mittelpunkt zum Einstellen des Unterdrucks drehbar gelagert ist. Auf dem Ring ist mindestens eine Markierung vorgesehen, wobei die Verstelleinheit derart mit dem Manometer synchronisiert ist, dass sich zumindest im Bereich zwischen dem unteren Grenzwert und dem oberen Grenzwert jeweils derjenige Unterdruck in dem Absaugkanal und somit auch auf dem Manometer einstellt, der durch die Markierung des Rings auf der Skala angezeigt ist. Somit wird eine doppelte Nutzung der Skala erreicht, nämlich zum einen zum Anzeigen des aktuellen Unterdrucks durch das Manometer und zum anderen zum Einstellen des gewünschten Unterdrucks über die Verstelleinheit durch die Bedienperson. Insbesondere ist es somit möglich, dass die Bedienperson auf besonders einfache Weise den gewünschten Unterdruck einstellen kann. Die Bedienperson muss, anders als bei bekannten Saugern mit einem Nadelventil, nicht aufwändig durch iteratives Probieren den Drehknopf des Saugers solange verstellen, bis sich irgendwann der gewünschte Unterdruck einstellt.

Vorzugsweise ist zumindest ein Teil des Absaugkanals durch einen Absaugschlauch ausgebildet, der auf einfache Weise in die Körperöffnung eingebracht werden kann. Zum Antreiben der Vakuumpumpe ist insbesondere ein elektrischer Motor vorgesehen.

Die Markierung des Rings ist vorzugsweise in Form einer Vertiefung in der Oberfläche des Rings ausgebildet, so dass diese Vertiefung nicht nur zur Markierung dient, sondern auch der Ring manuell durch das Auflegen eines Fingers in diese Vertiefung leicht gedreht werden kann. So wird eine besonders einfache Bedienung erreicht. Zusätzlich oder alternativ kann die Markierung auch in Form einer von der Farbe des Rings abweichenden, andersfarbigen Kennzeichnung erfolgen.

Der Ring ist insbesondere derart ausgebildet, dass er um 210° drehbar gelagert ist. Entsprechend ist auch die Skala derart ausgebildet, dass zwischen dem unteren Grenzwert und dem oberen Grenzwert ein Winkel zwischen 210° angeordnet ist.

Weitere Merkmale und Vorteile der Erfindung ergeben sich aus der folgenden Beschreibung, die die Erfindung anhand von Ausführungsbeispielen im Zusammenhang mit den beigefügten Figuren näher erläutert.

Es zeigen:
- Fig.1: eine schematische Darstellung einer Vorrichtung zum Absaugen von Flüssigkeiten und/oder Partikeln aus Körperöffnungen;
- Fig.2: eine schematische Darstellung einer Bedieneinheit der Vorrichtung nach Fig.1,
- Fig.3: eine schematische, perspektivische Darstellung einer Nebenlufteinstelleinheit der Vorrichtung nach den Figuren 1 und 2;
- Fig.4: eine Draufsicht auf die Nebenlufteinstelleinheit nach Figur 3 in einer ersten Stellung;
- Fig.5: eine Draufsicht auf die Nebenlufteinstelleinheit nach Figur 3 in einer zweiten Stellung; und
- Fig.6: eine Draufsicht auf die Nebenlufteinstelleinheit nach Figur 3 in einer dritten Stellung;

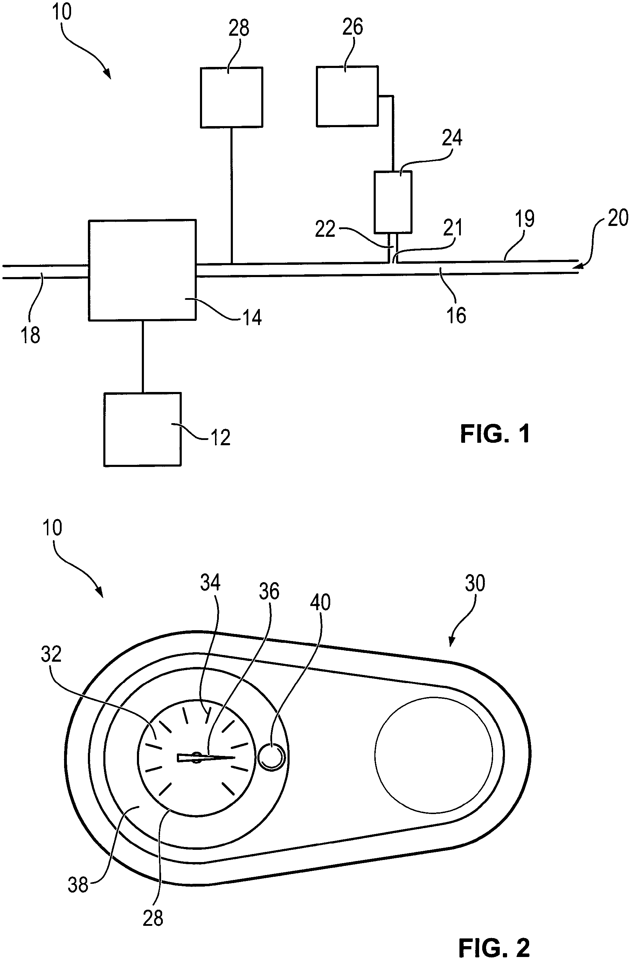

In Fig.1 ist eine schematische Darstellung einer Vorrichtung 10 zum Absaugen von Flüssigkeiten und/oder Partikeln aus Körperöffnungen vorgesehen, die üblicherweise im medizinischen Bereich, beispielsweise in Kliniken, Arztpraxen, Pflegeheimen und/oder bei der Heimpflege eingesetzt wird und häufig auch als Sekretsauger bezeichnet wird.

Die Vorrichtung 10 umfasst eine über einen elektrischen Motor 12 angetriebene Pumpe (Kompressor) 14, über die in einem Absaugkanal 16 ein Unterdruck erzeugbar ist, über den die Flüssigkeit und/oder die Partikel aus den Körperöffnungen abgesaugt werden. Die Energieversorgung des Motors kann hierbei über ein an das elektrische Stromnetz anschließbares Netzteil und/oder über Akkumulatoren erfolgen, so dass die Vorrichtung 10 wahlweise sowohl für den stationären Einsatz als auch für den mobilen Einsatz verwendbar ist. Ferner ist ein Abluftkanal 18 vorgesehen, über den die über den Absaugkanal 16 von der Pumpe abgesaugte Luft wieder abgeführt wird, nachdem die Flüssigkeit und/oder die Partikel aus dem Kanal entfernt und in einen nicht dargestellten Vorratsbehälter gefüllt wurden.

Zumindest ein Teilbereich des Absaugkanals 16 ist durch einen Absaugschlauch 19 ausgebildet, der an seinem der Pumpe 14 abgewandten Ende eine Absaugöffnung 20 aufweist, durch die die Flüssigkeit und/oder die Partikel eingesaugt werden. Ein solcher Absaugschlauch 19 lässt sich auf einfache Weise in die entsprechende Körperöffnung einführen, so dass die Flüssigkeit und/oder die Partikel zuverlässig abgesaugt werden können und eine einfache, verletzungsfreie und komfortable Handhabung möglich ist.

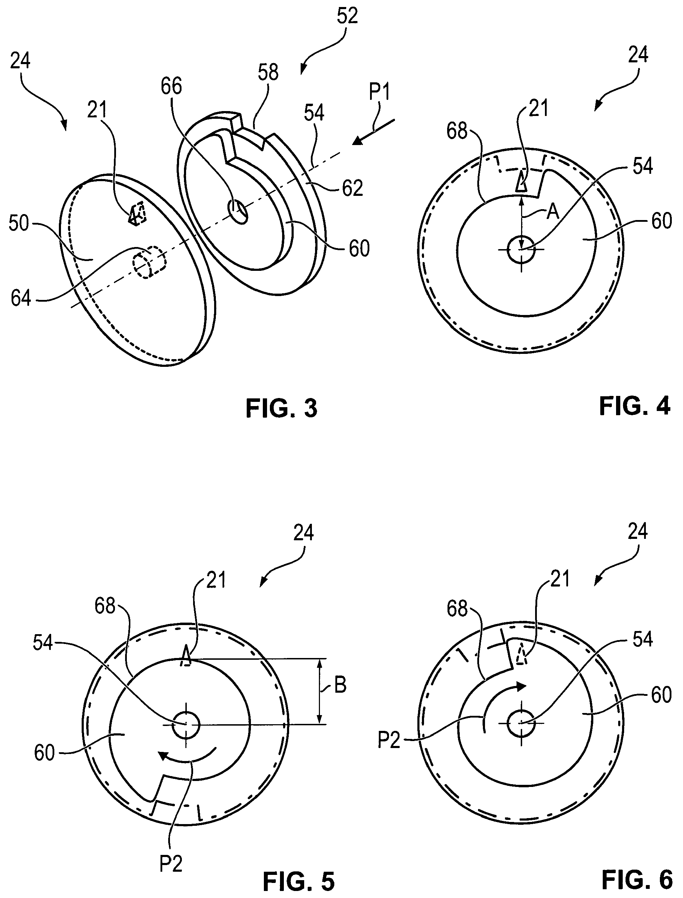

In dem Absaugkanal 16 ist eine Nebenluftöffnung 21 vorgesehen, über die durch einen Nebenluftkanal 22 herangeführte Nebenluft dem Absaugkanal 16 zugeführt werden kann. Der Volumenstrom dieser zugeführten Nebenluft ist über eine Nebenlufteinstelleinheit 24 einstellbar. Die Nebenlufteinstelleinheit 24 ist in Zusammenhang mit den Figuren 3 bis 6 noch näher beschrieben.

Über den Volumenstrom der Nebenluft lässt sich der in dem Absaugkanal 16 herrschende Unterdruck und somit die Saugleistung der Vorrichtung 10 einstellen. Je größer der zugeführte Volumenstrom der Nebenluft ist, umso geringer ist der Unterdruck.

Ferner ist ein Manometer 28 vorgesehen, dass den jeweils im Absaugkanal 16 herrschenden Unterdruck anzeigt, so dass dieser auf einfache Weise von der Bedienperson überwacht werden kann. Die Nebenlufteinstelleinheit 24 stellt den Volumenstrom der Nebenluft durch Verändern des für den Volumenstrom der Nebenluft zur Verfügung stehenden Querschnitts des Nebenluftkanals 22 und/oder der Öffnung 21 ein.

In Fig.2 ist eine schematische Darstellung eines Bedienfeldes 30 der Vorrichtung 10 gezeigt. Das Manometer 28 weist einen Anzeigebereich 32 auf, in dem eine kreissektorförmige Skala 34 angeordnet ist. Über einen Zeiger 36 wird auf dieser Skala 34 der jeweils im Absaugkanal 16 herrschende Unterdruck angezeigt. Die Skala 34 erstreckt sich insbesondere über einen Winkelbereich von 210°. Alternativ kann die Skala 34 auch über einen größeren oder kleineren Winkelbereich ausgebildet sein, insbesondere kann die Skala 34 auch kreisförmig sein. Der maximal mit Hilfe der Vorrichtung einstellbare Unterdruck beträgt insbesondere -80 kPa und wird auch als oberer Grenzwert bezeichnet. Der minimal einstellbare Unterdruck entspricht insbesondere 0 kPa, d.h. dass der atmosphärische Druck herrscht und somit kein Unterdruck vorliegt. Entsprechend erstreckt sich die Skala 34 insbesondere mindestens von 0 kPa bis -80 kPa.

Die Verstelleinheit 26 weist einen Ring 38 auf, der konzentrisch zu der Skala 34 angeordnet ist, d.h. dass der Mittelpunkt des Rings 38 mit demjenigen Punkt, um den der Zeiger 36 drehbar gelagert ist, zusammenfällt. Der Ring 38 ist ebenfalls um diesen Mittelpunkt drehbar. Auf der Oberfläche des Rings 38 ist eine Vertiefung 40 vorgesehen, über die der Ring 38 auf einfache Weise von der Bedienperson gedreht werden kann und über die eine Markierung ausgebildet ist. Ferner hat diese Vertiefung 40 eine von der Farbe der Oberfläche des Rings 38, in der die Vertiefung 40 ausgebildet ist, abweichende Farbe.

Der Ring 38 und die Nebenlufteinstelleinheit 24 sind derart aufeinander synchronisiert, dass bei einem Drehen des Rings 38 um jeweils den gleichen Winkel sich der von der Nebenlufteinstelleinheit 24 freigegebene Querschnitt des Volumenstroms der Nebenluft derart verändert, dass ein linearer Zusammenhang zwischen dem Unterdruck und dem Drehwinkel der Verstelleinheit 26, insbesondere des Rings 38, besteht. Ferner sind das Manometer 28 und der Ring 38 derart aufeinander abgestimmt, dass sich jeweils derjenige Unterdruck einstellt, der durch die Vertiefung 40 des Rings auf der Skala 34 des Manometers 28 angezeigt wird, so dass der gewünschte Unterdruck auf einfache Weise durch die Bedienperson eingestellt werden kann. Durch das Synchronisieren des Rings 38 und des Manometers 28 wird eine besonders einfache intuitive Bedienung der Vorrichtung 10 erreicht. Ferner muss somit, anders als bei bekannten Saugern, zum Einstellen des Unterdrucks die Absaugöffnung 20 nicht verschlossen werden.

Die Nebenlufteinstelleinheit 24 weist insbesondere eine Blende und/oder einen Schieber auf, der bei einem Drehen des Rings 38 den Querschnitt der Öffnung 21 und/oder den Querschnitt des Nebenluftkanals 22 teilweise oder auch vollständig verschließt und somit den für den Volumenstrom der Nebenluft zur Verfügung stehenden Strömungsquerschnitt einstellt. Hierbei wird der Schieber beim Drehen des Rings 38 um 1° jeweils um den gleichen Verstellweg verschoben, so dass sich bei einem dreieckigen Querschnitt der Öffnung 21 bzw. des Nebenluftkanals 22 und einem schneckenförmigen Querschnitt der Blende bzw. des Schiebers ein linearer Zusammenhang zwischen dem Drehwinkel des Rings 38 und dem Unterdruck und somit der Saugleistung der Vorrichtung 10 ergibt.

Alternativ kann anstelle eines Schiebers bzw. einer Blende auch ein Ventil mit einer entsprechenden Kennlinie verwendet werden.

Ferner ist es alternativ möglich, dass anstelle eines Rings 38 ein Drehknopf vorgesehen ist, über den der jeweilige Unterdruck einstellbar ist. Dieser Drehknopf ist insbesondere benachbart zu dem Manometer 28, aber nicht konzentrisch angeordnet. Auch in diesem Fall ergibt sich der Vorteil, dass zum Verstellen des Unterdrucks um den gleichen Wert jeweils die gleiche Drehung des Drehknopfes benötigt wird und somit eine einfache Bedienung erreicht ist. Allerdings ist es nicht möglich, den gewünschten Unterdruck über die Skala 34 des Manometers 28 einzustellen.

In Fig.3 ist eine schematische, perspektivische Darstellung der Nebenlufteinstelleinheit 24 in einer Explosionsdarstellung gezeigt. Fig.4 zeigt eine Draufsicht auf die Nebenlufteinstelleinheit 24 nach Fig.3 in einer ersten Stellung mit Blickrichtung in Richtung des Pfeiles P1 nach Fig.3.

Die Nebenlufteinstelleinheit 24 umfasst eine ortsfest angeordnete Scheibe 50, in der die Öffnung 21, durch die die Nebenluft dem Absaugkanal 18 zuführbar ist, ausgebildet ist. Die Öffnung 21 hat den Querschnitt eines Dreiecks, wobei es sich bei diesem Dreieck insbesondere um ein spitzwinkliges, gleichschenkliges Dreieck handelt.

Ferner umfasst die Nebenlufteinstelleinheit 24 eine relativ zur Scheibe 50 drehbar angeordnete Dreheinheit 52, die eine Blende 60 sowie eine fest mit der Blende 60 verbundene Scheibe 62 umfasst. Die Blende 60 und die Scheibe 62 sind insbesondere einteilig ausgebildet.

Die Blende 60 ist insbesondere schneckenförmig, d. h. in Form einer Kurvenscheibe, ausgebildet. Die Kontur 68 der Blende ist somit insbesondere spiralförmig.

Die Scheibe 50 und die Dreheinheit 52 sind insbesondere konzentrisch zueinander angeordnet, so dass sie um eine gemeinsame Mittelachse 54 drehbar gelagert sind. An der Scheibe 50 ist hierzu insbesondere ein Bolzen 64 vorgesehen, der in eine komplementär ausgebildete Aussparung 66 der Dreheinheit 52 eingreift.

In der Scheibe 62 der Dreheinheit 52 ist eine Aussparung 58 vorgesehen, so dass die durch die Öffnung 21 strömende Nebenluft durch diese Aussparung 58 strömen kann.

Die Schneckenform der Blende 60 ist derart ausgebildet, dass der Abstand A ihrer Kontur 68 zu der Mittelachse 54 in Abhängigkeit des Drehwinkels der Blende 60 variiert. Bei der in Fig.4 gezeigten ersten Stellung ist die Blende 60 derart gedreht, dass die Blende 60 die Öffnung 21 nicht verschließt und der gesamte Querschnitt der Öffnung 21 von der Nebenluft durchströmt werden kann. Somit wird bei dieser ersten Stellung die maximale Nebenluft dem Absaugkanal 18 zugeführt, so dass sich der untere Grenzwert des Unterdrucks einstellt. Wird die Dreheinheit 52 und somit die Blende 60 im Uhrzeigersinn in Richtung des Pfeiles P2 gedreht, so verdeckt die Blende 60 mit zunehmendem Drehwinkel relativ zur ersten Stellung die Öffnung 21 zunehmend, so dass der für die Nebenluft zur Verfügung stehende Strömungsquerschnitt abnimmt und somit der Volumenstrom der Nebenluft ebenfalls geringer wird. Somit stellt sich ein höherer Unterdruck ein. Bei der in Fig.5 gezeigten zweiten Stellung ist die Dreheinheit 52, und somit die Blende 60, soweit gedreht, dass die Blende 60 aufgrund des größeren Abstandes B der Kontur 68 der Blende 60 zu der Mittelachse 54 die Öffnung 21 nun teilweise bedeckt.

Wird die Blende 60 weiter in Richtung des Pfeiles P2 gedreht, so bedeckt sie in der in Fig.6 gezeigten dritten Stellung die Öffnung 21 vollständig, so dass durch die Öffnung 21 keine Nebenluft mehr strömen kann und sich der obere Grenzwert des Unterdrucks einstellt.

Durch die Dreiecksform der Öffnung 21 und die schneckenförmige Ausbildung der Blende 60 wird erreicht, dass ein linearer Zusammenhang zwischen dem Drehwinkel der Blende 60 und dem sich einstellenden Unterdruck im Absaugkanal 18 herrscht. Somit wird der Unterdruck bei einer Drehung der Blende 60 um den gleichen Winkel jeweils um den gleichen Wert verändert, so dass sich bei einer entsprechenden Synchronisation zwischen dem Ring 38 der Verstelleinheit 26 und der Dreheinheit 52 der gewünschte lineare Zusammenhang zwischen der Stellung der Verstelleinheit 26 und dem sich einstellenden Unterdruck ergibt.

### Bezugszeichenliste

- 10: Vorrichtung
- 12: Motor
- 14: Pumpe
- 16: Absaugkanal
- 18: Abluftkanal
- 19: Absaugschlauch
- 20: Absaugöffnung
- 21: Öffnung
- 22: Nebenluftkanal
- 24: Nebenlufteinstelleinheit
- 26: Verstelleinheit
- 28: Manometer
- 30: Bedienfeld
- 32: Anzeigebereich
- 34: Skala
- 36: Zeiger
- 38: Ring
- 40: Vertiefung
- 50: Scheibe
- 52: Dreheinheit
- 54: Achse
- 58: Aussparung
- 60: Blende
- 62: Scheibe
- 64: Bolzen
- 66: Aussparung
- 68: Kontur
- A, B: Abstand
- P1: Blickrichtung
- P2: Drehrichtung

## Patentansprüche

1. Vorrichtung zum Absaugen von Flüssigkeiten und/oder Partikeln aus Körperöffnungen,
mit einem Absaugkanal (16), durch den die Flüssigkeit und/oder die Partikel absaugbar ist,
einer Vakuumpumpe (14) zum Erzeugen eines Unterdrucks in dem Absaugkanal (16) zum Absaugen der Flüssigkeit und/oder der Partikel, wobei der Absaugkanal (16) eine Öffnung (21) zum Zuführen von Nebenluft zum Einstellen des Unterdrucks aufweist,
einer Nebenlufteinstelleinheit (24) zum Einstellen des Volumenstroms der dem Absaugkanal (16) zugeführten Nebenluft, und
mit einer manuell betätigbaren Verstelleinheit (26) zum Einstellen der Nebenlufteinstelleinheit (24),
wobei zumindest im Bereich zwischen einem unteren Grenzwert des Unterdrucks und einem oberen Grenzwert des Unterdrucks das Verstellen der Verstelleinheit (26) um jeweils einen gleichen voreingestellten Verstellweg jeweils die gleiche Änderung des Unterdrucks bewirkt, und
**dadurch gekennzeichnet, dass** die Öffnung (21) einen dreieckigen Querschnitt hat, und dass die Nebenlufteinstelleinheit (24) eine schneckenförmige Blende zum Begrenzen des Querschnitts hat.

2. Vorrichtung (10) nach Anspruch 1, **dadurch gekennzeichnet, dass** die Verstelleinheit (26) stufenweise verstellbar ist, und dass zumindest im Bereich zwischen dem unteren Grenzwert und dem oberen Grenzwert das Verstellen der Verstelleinheit (26) um jeweils eine Stufe jeweils die gleiche Änderung des Unterdrucks bewirkt.

3. Vorrichtung (10) nach Anspruch 1 oder 2, **dadurch gekennzeichnet, dass** die Nebenlufteinstelleinheit (24) ein Ventil umfasst.

4. Vorrichtung (10) nach einem der vorhergehenden Ansprüche, **dadurch gekennzeichnet, dass** Nebenlufteinstelleinheit (24) eine Blende zum Verschließen mindestens eines Teilbereichs eines Nebenluftkanals (22) zum Zuführen der Nebenluft und/oder mindestens eines Teilbereichs der Öffnung (21) zum Zuführen der Nebenluft umfasst.

5. Vorrichtung (10) nach einem der vorhergehenden Ansprüche, **dadurch gekennzeichnet, dass** ein Manometer (28) zum Anzeigen des jeweils aktuellen Unterdrucks vorgesehen ist.

6. Vorrichtung (10) nach Anspruch 5, **dadurch gekennzeichnet, dass** das Manometer (28) eine kreissektorförmige Skala (34) und einen um den Mittelpunkt der kreissektorförmigen Skala (34) drehbar gelagerten Zeiger (36) zum Anzeigen der aktuellen Unterdrucks auf der Skala (34) umfasst, dass die Verstelleinheit (26) einen Ring (38) umfasst, dessen Mittelpunkt mit dem Mittelpunkt des Skala (34) zusammenfällt, dass der Ring (38) um seinen Mittelpunkt zum Einstellen des Unterdrucks drehbar gelagert ist, dass auf dem Ring (38) mindestens eine Markierung vorgesehen ist, und dass die Verstelleinheit (26) derart auf das Manometer (28) synchronisiert ist, dass sich zumindest im Bereich zwischen dem unteren Grenzwert und dem oberen Grenzwert jeweils derjenigen Unterdruck einstellt, der durch die Markierung (40) des Rings (38) auf der Skala (34) angezeigt ist.

7. Vorrichtung (10) nach Anspruch 6, **dadurch gekennzeichnet, dass** die Markierung (40) in Form einer Vertiefung in der Oberfläche des Rings (38) ausgebildet ist.

8. Vorrichtung (10) nach Anspruch 6 oder 7, **dadurch gekennzeichnet, dass** zumindest die sichtbare Oberfläche des Rings (38) eine erste Farbe aufweist, und dass die Markierung (40) eine von der ersten Farbe verschiedene zweite Farbe hat.

9. Vorrichtung (10) nach einem der Ansprüche 6 bis 8, **dadurch gekennzeichnet, dass** der Ring (38) um einen Winkel im Bereich zwischen 90° und 270° drehbar ist.

10. Vorrichtung (10) nach einem der vorhergehenden Ansprüche, **dadurch gekennzeichnet, dass** die Vorrichtung (10) einen Absaugschlauch (19) umfasst, durch den zumindest ein Teil des Absaugkanals (16) ausgebildet ist.

11. Vorrichtung (10) nach einem der vorhergehenden Ansprüche, **dadurch gekennzeichnet, dass** ein Motor (12) zum Antreiben der Vakuumpumpe (14) vorgesehen ist.

12. Vorrichtung (10) nach einem der vorhergehenden Ansprüche, **dadurch gekennzeichnet, dass** der obere Grenzwert des Unterdrucks einen Wert zwischen -70 kPa und -90 kPa hat.

13. Vorrichtung (10) nach einem der vorhergehenden Ansprüche, **dadurch gekennzeichnet, dass** der untere Grenzwert des Unterdrucks einen Wert zwischen -0 kPa und -10 kPa hat.

## Claims

1. Device for suction of liquids and/or particles from body orifices,
comprising a suction channel (16) through which the liquid and/or the particles can be sucked,
a vacuum pump (14) for generating a negative pressure in the suction channel (16) for suction of the liquid and/or the particles, wherein the suction channel (16) comprises an opening (21) for supplying additional air for adjusting the negative pressure,
an additional air adjustment unit (24) for adjusting the volume flow of the additional air supplied to the suction channel (16), and
comprising a manually operable adjustment unit (26) for adjusting the additional air adjustment unit (24),
wherein at least in the range between a lower limit value of the negative pressure and an upper limit value of the negative pressure the adjustment of the adjustment unit (26) in each case by a same preset adjustment path effects in each case the same change in the negative pressure, and **characterised in that** the opening (21) has a triangular cross-section
and **in that** the additional air adjustment unit (24) has a helical cover plate for limiting the cross-section.

2. Device (10) as claimed in claim 1, **characterised in that** the adjustment unit (26) is adjustable in a stepwise manner and **in that** at least in the range between the lower limit value and the upper limit value, the adjustment of the adjustment unit (26) in each case by one step effects in each case the same change in the negative pressure.

3. Device (10) as claimed in claim 1 or 2, **characterised in that** the additional air adjustment unit (24) comprises a valve.

4. Device (10) as claimed in any one of the preceding claims, **characterised in that** the additional air adjustment unit (24) comprises a cover plate for closing at least a portion of an additional air channel (22) for supplying the additional air and/or at least a portion of the opening (21) for supplying the additional air.

5. Device (10) as claimed in any one of the preceding claims, **characterised in that** a manometer (28) is provided for displaying the respectively current negative pressure.

6. Device (10) as claimed in claim 5, **characterised in that** the manometer (28) comprises a circular sector-shaped scale (34) and a pointer (36) mounted so as to be able to rotate about the centre point of the circular sector-shaped scale (34) for displaying the current negative pressure on the scale (34), **in that** the adjustment unit (26) comprises a ring (38), the centre point of which coincides with the centre point of the scale (34), **in that** the ring (38) is mounted so as to be able to rotate about its centre point for adjusting the negative pressure, **in that** on the ring (38) at least one marking is provided and **in that** the adjustment unit (26) is synchronised to the manometer (28) such that at least in the range between the lower limit value and the upper limit value in each case the particular negative pressure which is displayed by the marking (40) of the ring (38) on the scale (34) is adjusted.

7. Device (10) as claimed in claim 6, **characterised in that** the marking (40) is formed in the manner of a recess in the surface of the ring (38).

8. Device (10) as claimed in claim 6 or 7, **characterised in that** at least the visible surface of the ring (38) has a first colour and **in that** the marking (40) has a second colour which is different from the first colour.

9. Device (10) as claimed in any one of claims 6 to 8, **characterised in that** the ring (38) is rotatable about an angle in the range between 90° and 270°.

10. Device (10) as claimed in any one of the preceding claims, **characterised in that** the device (10) comprises a suction hose (19), by means of which at least a part of the suction channel (16) is formed.

11. Device (10) as claimed in any one of the preceding claims, **characterised in that** a motor (12) is provided for driving the vacuum pump (14).

12. Device (10) as claimed in any one of the preceding claims, **characterised in that** the upper limit value of the negative pressure has a value between -70 kPa and -90 kPa.

13. Device (10) as claimed in any one of the preceding claims, **characterised in that** the lower limit value of the negative pressure has a value between -0 kPa and -10 kPa.

## Revendications

1. Dispositif permettant d'aspirer des liquides et/ou des particules émanant d'orifices corporels,
comprenant un canal d'aspiration (16) par lequel le liquide et/ou les particules peuvent être aspirés,
une pompe à vide (14) conçue pour produire une dépression dans le canal d'aspiration (16) pour aspirer le liquide et/ou les particules, le canal d'aspiration (16) présentant une ouverture (21) permettant d'amener de l'air additionnel pour établir la dépression,
une unité de régulation d'air additionnel (24) destinée à réguler le débit volumétrique de l'air additionnel amené au canal d'aspiration (16), et
une unité de réglage (26) à actionnement manuel conçue pour régler l'unité de régulation d'air additionnel (24),
le réglage de l'unité de réglage (26) impliquant la même modification de la dépression à chaque même course de réglage préétablie, au moins dans la plage comprise entre une valeur seuil inférieure de la dépression et une valeur seuil supérieure de la dépression,
**caractérisé en ce que** l'ouverture (21) présente une section transversale triangulaire,
et **en ce que** l'unité de régulation de l'air additionnel (24) présente un obturateur hélicoïdal permettant de délimiter la section transversale.

2. Dispositif (1) selon la revendication 1, **caractérisé en ce que** l'unité de réglage (26) est réglable graduellement, et **en ce que** le réglage de l'unité de réglage (26) implique la même modification de la dépression à chaque étape, au moins dans la plage comprise entre la valeur seuil inférieure et la valeur seuil supérieure.

3. Dispositif (10) selon la revendication 1 ou 2, **caractérisé en ce que** l'unité de régulation de l'air (24) comprend une soupape.

4. Dispositif (10) selon l'une quelconque des revendications précédentes, **caractérisé en ce que** l'unité de régulation de l'air additionnel (24) comprend un obturateur permettant de fermer au moins une partie d'un canal d'air additionnel (22) destiné à amener l'air additionnel et/ou moins d'une partie de l'ouverture (21) destinée à amener l'air additionnel.

5. Dispositif (10) selon l'une quelconque des revendications précédentes, **caractérisé en ce qu'**un manomètre (28) est destiné à indiquer la dépression respectivement actuelle.

6. Dispositif (10) selon la revendication 5, **caractérisé en ce que** le manomètre (28) comprend un cadran (34) en forme de secteur de cercle et une aiguille (36) montée de manière à pouvoir tourner autour du centre du cadran (34) en forme d'arc de cercle et destinée à indiquer la dépression actuelle sur le cadran (34), **en ce que** l'unité de réglage (26) comprend une bague (38) dont le centre coïncide avec le centre du cadran (34), **en ce que** la bague (38) est montée de manière à pouvoir tourner autour de son centre pour régler la dépression, **en ce qu'**au moins un marquage est prévu sur la bague (38), et **en ce que** l'unité de réglage (26) est synchronisée sur le manomètre (28), de telle sorte que respectivement la dépression qui est indiquée par le marquage (40) de la bague (38) sur le cadran (34) est réglée au moins dans la plage comprise entre la valeur seuil inférieure et la valeur seuil supérieure.

7. Dispositif (10) selon la revendication 6, **caractérisé en ce que** le marquage (40) est réalisé sous la forme d'un creux dans la surface de la bague (38).

8. Dispositif (10) selon la revendication 6 ou 7, **caractérisé en ce qu'**au moins la surface visible de la bague (38) présente une première couleur, et **en ce que** le marquage (40) présente une deuxième couleur différente de la première couleur.

9. Dispositif (10) selon l'une quelconque des revendications 6 à 8, **caractérisé en ce que** la bague (38) peut tourner selon un angle dans la plage comprise entre 90° et 270°.

10. Dispositif (10) selon l'une quelconque des revendications précédentes, **caractérisé en ce que** le dispositif (10) comprend un tuyau d'aspiration (19) par lequel au moins une partie du canal d'aspiration (16) est formée.

11. Dispositif (10) selon l'une quelconque des revendications précédentes, **caractérisé en ce qu'**un moteur (12) est destiné à entraîner la pompe à vide (14).

12. Dispositif (10) selon l'une quelconque des revendications précédentes, **caractérisé en ce que** la valeur seuil supérieure de la dépression présente une valeur comprise entre -70 kPa et -90 kPa.

13. Dispositif (10) selon l'une quelconque des revendications précédentes, **caractérisé en ce que** la valeur seuil inférieure de la dépression présente une valeur comprise entre -0 kPa et -10 kPa.
